# EUROPEAN PATENT SPECIFICATION

(11) **EP 1 632 258 B1**
(45) Date of publication and mention of the grant of the patent: **16.02.2011**
(21) Application number: 05018089.2
(22) Date of filing: 19.08.2005
(51) Int. Cl.: A61L 31/04

(54) **Intravascular, indwelling instrument having a peptide fixed thereto**
Intravaskuläres Verweilinstrument mit Peptid-Beschichtung
Instrument intravasculaire à demeure ayant un revêtement de peptide

(30) Priority: 03.09.2004 JP 2004257286
(43) Date of publication of application: 08.03.2006
(73) Proprietor: TERUMO KABUSHIKI KAISHA, Tokyo (JP)
(72) Inventor: Kanamaru, Takeshi, Ashigarakami-gun, Kanagawa 259-0151 (JP); Fujita, Yotaro, Ashigarakami-gun, Kanagawa 259-0151 (JP)
(74) Representative: Casalonga, Axel

(56) References cited:
- WO-A-00/40278
- WO-A-2004/080397
- US-A- 5 670 161
- US-A1- 2004 106 987
- US-A1- 2004 161 443

## Description

This invention relates to an intravascular, indwelling instrument which acts to reduce or inhibit a blood flow not to be maintained in a blood vessel under pathologic conditions and also to promote the organization of the pathologic or diseased blood vessel.

For blood vessel diseases, there are known an aneurysm, a varicosity, artery obstruction and thrombophlebitis (blood vessels having diseases being hereinafter referred to as "diseased blood vessel"). Of these, the aneurysm is a disease wherein because of a strong pressure (blood pressure) invariably exerted on side walls of an arterial vessel, a weak part or area of the walls swells or dilates, and "bump", i.e. "swelling", is formed at the part. The swelling has such a shape that the side walls of the blood vessel spherically swells or dilates with a neck being at the inlet (opening) of the aneurysm formed at the side walls of the blood vessel. Normally, no specific symptom is involved and the patient feels no pain. Secondary troubles may be brought about depending on the shapes of the swelling (a shape and size of the opening and a shape and size of the swollen and dilated portion). In the chest, for instance, the aneurysm grown to a large size compresses peripheral tissues, eventually leading to huskiness, no progressing of food through the throat, and occurrence of blood-streaked sputum. Alternatively, the bloodstream flowing into the swelling imparts an abnormal pressure to the inner walls thereof, thus leading to the breakage of the swelling. As a result, bleeding from the broken site of the swelling takes place, with some possibility that the patient suffers from the poverty of blood and shock, or a serious disease such as brain hemorrhage may be caused. Thus, in order to suppress the secondary trouble caused by the swelling, it is necessary, prior to the swelling being grown to a given diameter or size, to reduce or inhibit an abnormal blood flow into the swelling while keeping the normal blood flow in blood vessels and cure the aneurysm. The term "abnormal blood flow" used herein means a blood flow passing through a diseased blood vessel (including the above-mentioned swelling), and the term "normal blood flow" means a blood flow passing through normal vessels. Depending on the purpose, the term "blood flow to be maintained" used herein is defined as a blood flow whose stream or flow should be maintained (managed), and the term "blood flow not to be maintained" defined as a blood flow unnecessary for maintenance of the flow. In general, for example, with the curing of aneurysm, the "blood flow not to be maintained" means "abnormal blood flow", and the "blood flow to be maintained" means "normal blood flow". In this connection, however, as will be described hereinafter, where an anticancer drug is passed into a pathologic site for curing liver cancer, the "normal blood flow" is "blood flow not to be maintained", and the "abnormal blood flow" is "blood flow to be maintained".

For curing of aneurysm, there have been hitherto known several surgical operations including a method wherein a diseased blood vessel in the vicinity of aneurysm is surgically removed, followed by exchange with an artificial blood vessel, and a so-called clipping method wherein the swollen portion of aneurysm is pinched with a clip from outside of the blood vessel to inhibit the abnormal blood flow (blood flow not to be maintained) from entering into the aneurysm. However, the surgical operation places a great burden on patient and thus, is unsuited as a curing method for aneurysm-bearing patients most of which are aged persons.

In recent years, a new curing method (aneurysm embolization) is being under development wherein a catheter is introduced into a blood vessel percutaneously such as from a crural area, advancing the tip of the catheter under radioscopy to a target site, e.g. a position of the aneurysm of the blood vessel in the brain, and supplying and filling a coil-shaped or particulate embolizing substance inside the aneurysm through a lumen formed within the catheter thereby inhibiting a blood flow not to be maintained from entering into the aneurysm and promoting the organization thereof. This curing method is advantageous in that the risk of the surgical operation and the burden on the part of a patient can be significantly mitigated. On the other hand, however, where a coil-shaped embolizing substance is used in this method, a problem has been arisen in that a bloodstream enters into spaces established owing to imperfect filling of the space in the swelling with the coil-shaped embolizing substance, and the swelling becomes larger in size, resulting in bursting. In this connection, with a particulate embolizing substance, its size is smaller than that of the coil, enabling one to fill it within an aneurysm substantially in a space-free condition. Nevertheless, the particle size is so small that possible withdrawal from the aneurysm is facilitated. If an embolizing substance is so withdrawn or separated from the swelling as mentioned above and blocks up the blood vessel with the embolizing substance, there is a risk of causing downstream tissues to be subject to necrosis.

In the treatment according to an aneurysm embolizing technique using a coil-shaped embolizing substance, an instance is known in which endothelial conversion at the pathologic part has not been attained a half year after operation although depending on the size, shape or occurrence site of the swelling.

Under these circumstances in the art, for solving the problem on the withdrawal of an embolizing substance in the treatment using embolizing substances, there has been proposed a method wherein a stent is inserted into and placed in the vicinity of an aneurysm of a blood vessel, and an embolizing susbtance is filled within the aneurysm through an opening formed at a side wall of the stent in a cylindrical form (see, for example, Japanese Patent Laid-open No. 2003-250907). In this method, the stent formed with the opening at the side wall thereof should be so arranged that the opening at the side wall of the stent is coincident with the opening of the aneurysm. To this end, when inserted into and placed in a target site, the stent has to be rotated against the blood vessel to exactly determine the position along a peripheral direction. This presents a problem in that much time and labor is taken for the stent placement.

Another problem involved in the method of filling an embolizing substance through the opening at the side wall of a stent is a problem on a stent per se that is caused for achieving the intended purposes in use of the stent. The intended purposes in use of the stent are to maintain constricted portion of a blood vessel or other lumens in a dilated or extended condition. In this sense, hitherto, most frequently used metallic stent is so formed that a force thereof can be perpetually worked along a direction of extending the blood vessel, i.e. a radial force can be worked on the blood vessel. Document US 2004/106987 describes an intravascular instrument covered with a polymeric layer and containing a therapeutic agent possibly being a RGD peptide sequence and some stents composed of metallic filaments or metallic sheers on which the biodisintegrated polymer is coated. This may eventually cause intimal damages by the radial force upon the insertion and placement of the stent, or may cause chronic inflammation at the endomembrane upon long-term placement. Such intimal damages cause a decrease in function of endothelial cells, and streaming of smooth muscle cells toward the intima of blood cells and hyperproliferation. As a consequence, a problem on restenosis of the vessel arises. From the above standpoint, it is hard to say that the method of filling an embolizing substance through an opening formed at the side wall of the stent is the best one for curing aneurysm.

Stents used for preventing restenosis have been proposed (see, for example, Japanese Patent Laid-open No. 2 004-97810). These stents are formed of biocompatible materials or biodegradable polymer materials and contain, for the purpose of preventing restenosis, medicines capable of suppressing streaming and proliferation of smooth muscle cells or medicines for improving the function of vascular endothelial precursor cells. WO00/40278 describes some stent graft wherein the graft releases an agent which induces the in vivo adhesion of the stent to vessel walls. The graft portion around the stent may be composed of textile or polymer optionally containing peptides.

By the way, there have been known, up to now, several peptides that are capable of specific interaction with endothelial cell precursor substances in body fluids including tissues or blood. One of the factors as to why these peptides have the specific interaction with endothelial cell precursor substances is considered as follows: these peptides have such amino acid sequences similar to those amino acid sequences of proteins capable of interaction with integrin that is a kind of a cell attachment factor (see, for example, Jeffrey A. Hubbel and other three "BIO/TECHNOLOGY" (United States of America), June, 1991 Vol. 9, pp.568 to 572). US 2004/161443 relates to vehicles comprising melanocyte stimulating hormone (MSH) for its use in implantable materials such as coronary artery stents. MSH peptides may be coupled to RGD motifs via linkers like PEG. WO 2004/080397 discloses some stents able to release some therapeutic agents and possibly coated with several polymers regulating the rate of release of the therapeutic agent. This therapeutic agent may be an endothelialization facilitator like RGD peptide. US 5 670161 discloses a stent made of a copolymer of L-lactic acid and ε-caprolactone wherein peptides like Hirudin arc incorporated, either into the copolymer forming the stent or into the coating.

It is an object of the invention to provide an intravascular, indwelling instrument which is easy in insertion into and placement in a target site, and is capable of reducing or inhibiting blood flow not to be maintained from entering into an aneurysm irrespective of supply and filling of an embolizing substance whereby the organization of the aneurysm is facilitated. The term "target site" used herein means a site wherein in case where the intravascular, indwelling instrument is applied to a given diseased vessel, the instrument is to be placed for reducing or inhibiting the inflow of a blood flow not to be maintained, and especially with aneurysm, it means an inner wall of a normal blood vessel in the vicinity of an opening of the aneurysm.

The above object can be achieved, according to the invention, by intravascular, indwelling instruments defined in (1) to (8) below.
(1) An intravascular, indwelling instrument for placement in a blood vessel, including: a stent made of high polymeric material having a maintained blood flow contact face in contact with a blood flow to be maintained and a non-maintained blood flow contact face in contact with a blood flow not to be maintained; and a peptide fixed to all or part of the maintained blood flow contact face or the non-maintained blood flow contact face of the stent and having specific interaction with vascular endothelial precursor cells, wherein the peptide permits selective adsorption and adhesion of the vascular endothelial precursor cells to cover all or part of the maintained blood flow contact face or the non-maintained blood flow contact face of the stent with the vascular endothelial precursor cells thereby reducing or inhibiting the blood flow not to be maintained, wherein the peptide has an amino acid sequence which is one of Arg-Glu-Asp-Val (REDV) (sequence No. 1), Arg-Gly-Asp (RGD) (sequence No. 2) and Tyr-Ile-Gly-Ser-Arg (YIGSR) (sequence No. 3).
(2) The intavascular, indwelling instrument wherein the high polymer material consists of a biodegradable polymer.
(3) The intavascular, indwelling instrument as recited in (2) above, wherein the biodegradable polymer consists of at least one member selected from the group consisting of polylactic acid, polyglycolic acid, polycaprolactone, polyethylene succinate, polybutylene succinate, polyhydroxy butyrate, polymalic acid, poly-a-amino acid, collagen, laminim, heparan sulfate, fibronectin, vitronectin, chondroitin sulfate and hyaluronic acid, or a copolymer of monomers for the above-defined biodegradable polymers.
(4) The intavascular, indwelling instrument as recited in any one of (1) to (3) above, wherein the peptide is fixed through polyethylene glycol (hereinafter abbreviated as PEG) serving as a spacer.

The intravascular, indwelling instrument of the invention is precutaneously inserted into and placed in a target site of a diseased blood vessel and thus, has the effect of significantly reducing the risk involved in surgical operation and the burden imposed on patients.

The indwelling instrument has a peptide, which has specific interaction with vascular endothelial precursor cells, in such a way that all or part of the face of the instrument body in contact with a blood flow to be maintained and the face thereof in contact with a blood flow not to be maintained is covered with the vascular endothelial precursor cells, and has thus the effect of facilitating endothelial conversion of the covered area.

When the indwelling instrument is formed of a biodegradable polymer material and/or a biocompatible polymer, the thickening of an intima of a blood vessel as would be caused by the indwelling instrument being inserted into and placed in a target site can be prevented.

Further, where the indwelling instrument of the invention is applied for curing a diseased blood vessel such as an aneurysm, the cure is feasible without use of an embolizing substance, and thus, the invention has the effect of preventing vascular obstruction with the embolizing substance. On the other hand, where the indwelling instrument of the invention is applied for curing an aneurysm along with an embolizing substance in the form of coils or particles, the problem on the withdrawal of the embolizing substance can be solved, with a synergistic effect of promoting the organization of the swelling.
Fig. 1 is a side view showing a stent according to an embodiment of the invention;
Fig. 2 is a schematic view showing an intravascular, indwelling instrument (stent) wherein peptide is directly fixed on a surface thereof;
Fig. 3 is a schematic view showing an intravascular, indwelling instrument wherein peptide is fixed through spacer on the surface;
Fig. 4 is a photograph showing a morphology of a rabbit two days after having embedding a stent of Example 1 in a carotid artery of an intentionally aneurysm-induced rabbit;
Fig. 5 is a photograph showing a morphology of a rabbit two days after having embedding a stent of Comparative Example 1 in a carotid artery of an intentionally aneurysm-induced rabbit;
Fig. 6 is a photograph showing a morphology of a rabbit two days after having embedding a stent of Example 2 at a neck portion of a saccular aneurysm formation-induced rabbit;
Fig. 7 is a photograph showing a morphology of a rabbit two days after having embedding a stent of Comparative Example 2 at a neck portion of a saccular aneurysm formation-induced rabbit;
Fig. 8 is a schematic view showing an intravascular indwelling instrument of the invention placed at an opening of an aneurysm; and
Fig. 9 is a schematic view showing the disappearance of an aneurysm as a result of application of an intravascular indwelling instrument of the invention to the aneurysm.

An intravascular, indwelling instrument of the invention is now described in detail.

An indwelling instrument of the invention includes a stent having a maintained blood flow contact face in contact with a blood flow to be maintained and a non-maintained blood flow contact face in contact with a blood flow not to be maintained, and a peptide having specific interaction with vascular endothelial precursor cells. The peptide is fixed to all or part of the maintained blood flow contact face or the non-maintained blood flow contact face of the stent.

An intravascular, indwelling stent of the invention has such an effect that when the instrument is placed at a target site, part or all of the maintained blood flow contact face and the non-maintained blood flow contact face of the stent is covered with the vascular endothelial precursor cells thereby facilitating the endothelial conversion of the site. As a result, with an aneurysm, for example, while keeping a blood flow, to be maintained, passing through a normal blood vessel, passage of a blood flow, not to be maintained, into the aneurysm is reduced or inhibited, so that the aneurysm through which passes the blood flow not to be maintained becomes organized and is thus rendered harmless.

The instrument of the invention is not limited to application to aneurysms, but may be applicable to other diseased or pathogenic blood vessels which need to be reduced or inhibited in respect of a blood flow not to be maintained.

For instance, where the tip of a bifurcated blood vessel becomes necrotized, the indwelling instrument is inserted into and placed in at the bifurcation of the blood vessel so that the flow of a blood flow not to be maintained is reduced or inhibited, whereupon part or all of the maintained blood flow contact face and the non-maintained blood flow contact face of the body of the instrument is covered with vascular endothelial precursor cells, thereby facilitating the endothelial conversion at the site. Eventually, the inflow in amount of the blood flow into the necrotized vessel not to be maintained is reduced or inhibited, and thus, the necrotized vessel is organized and becomes harmless.

Another possible instance for the application is curing of a liver cancer. In this case, when a blood stream or flow at a bifurcated or divergent blood vessel through which a blood flow not to be maintained passes is reduced or inhibited, it becomes possible to effectively force an anticancer drug to be fed into a diseased blood vessel through which a blood flow to be maintained passes.

As stated hereinabove, the instrument of the invention is placed at a target site in a blood bifurcation where a blood flow to be maintained and a blood flow not to be maintained are bifurcated from each other so as to block up the blood vessel at an inlet thereof at a side in which the blood stream not to be maintained flows. In this way, the blood flow not to be maintained can be reduced in amount or inhibited from entering.

### <Body of intravascular, indwelling instrument>

The stent of the instrument according to the invention is now described in detail.

For the instrument body of the invention, mention is, for example, of a stent used as an artificial blood vessel or blood vessel prosthetic material. These may be percutaneously placed at a target site of a blood vessel in a pathologic condition without resorting to surgical operation. The use of the intravascular, indwelling instrument in this way is the best embodiment of the invention.

The intravascular, indwelling instrument according to the invention is described in detail based on preferred embodiments shown in the accompanying drawings.

### <Stent>

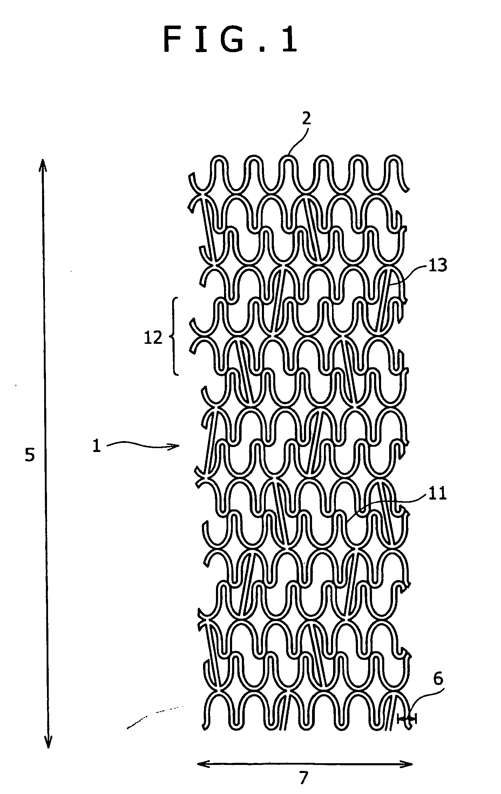

Fig. 1 is a side view showing an embodiment of a stent.

In the embodiment shown in Fig. 1, a stent body 1 is made of a linear member 2 and has an element 11 of a substantially rhombic shape having a notch in the inside thereof as a unit. A plurality of substantially rhombus-shaped elements 11 constitute an annular unit 12 by arranging and continuously combining the substantially rhombic shapes along a minor axis direction. The annular unit 12 is connected to an adjacent annular unit 12 through a linear connection member 13. In this way, a plurality of annular units 12 are continuously arranged along axes thereof in a partly combined condition. The stent body 1 is so arranged as set out above and is in the form of a cylinder having openings at ends thereof and extending along a length thereof between the ends. The stent 1 has notches of a substantially rhombic form, and thus, has such a structure as to be expanded and contracted along a radial direction of the cylindrical body through deformation at the notches. Hence, when the body 1 is placed in a blood vessel, its shape is kept as it is.

The stent used as an intravascular, indwelling instrument of the invention is not limited to the embodiment shown in the figure. Those stents, which are a cylindrical body having openings at ends thereof and extending along a length thereof between the ends and have, on sides surfaces thereof, a number of notches permitting communication between outer surfaces and inner surfaces, are likewise within the scope of the invention. In these types of stents, the deformation of the notches allows the stent to have a structure that is able to expand and contact along radial directions of the cylindrical body.

The term "face in contact with a blood flow to be maintained" when used for stent means an inner surface of stent, and the term "face in contact with a blood flow not to be maintained" means an outer surface of stent.

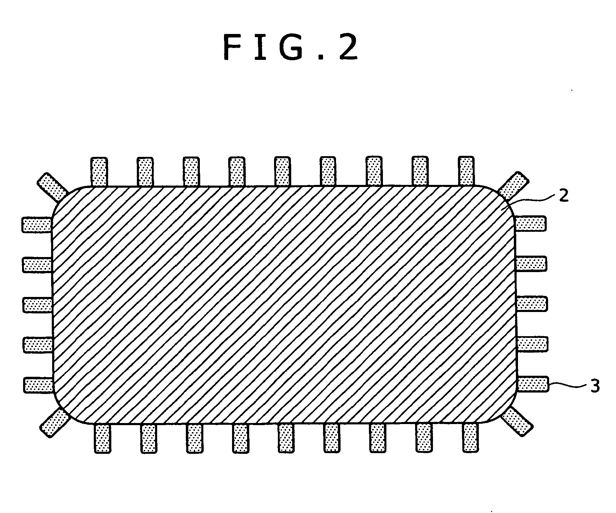

Specific examples of stents having such a structure as to permit expansion and contraction along radial directions include: a stent, as disclosed, for example, in Japanese Patent Laid-open Nos. Hei 9-215753 and Hei 7-529, wherein an elastic wire rod is coiled and a plurality of coils are connected in the form of a cylinder, and spaces established between adjacent elastic wire rods serve as a notch; a stent, as disclosed in Japanese Translations of PCT for Patent Nos. Hei 8-502428 and Hei 7-500272, wherein a elastic wire rod is bent in a zigzag form and a plurality of so shaped rods are connected in a cylindrical form, and spaces between elastic wire rods each act as a notch; a stent, as disclosed in Japanese Translations of PCT for Patent No. 2000-501328 and Japanese Patent Laid-open No. Hei 11-221288, wherein an elastic wire rod is bent in the form of a snaky flat ribbon and is wound about a mandrill in helix form to provide a cylindrical body in which spaces between adjacent elastic wire rods serve as a notch; a stent, as disclosed in Japanese Translations of PCT for Patent No. Hei 10-503676, having such a mesh-shaped structure wherein the shape of notches differs from that of the stent shown in Fig. 1 and is in a meander pattern; and a stent, as disclosed in Japanese Translations of PCT for Patent No. Hei 8-507243, wherein a plate member is coiled to provide a cylindrical body. in which a space established between adjacent spiral portions serves as a notch. Alternatively, in Japanese Patent Publication No. Hei 4-68939, mention is made of a cylinder-shaped stent having a plurality of structures including a stent obtained by spirally coiling an elastic plate member in a cylindrical form with a space between adjacent coiled portions serving as a notch, and a stent obtained by braiding an elastic wire rod in the form of a cylinder with a space between adjacent spiral portions being provided as a notch. These stents may be applied to as a body for the intravascular indwelling instrument of the invention. Besides, for use as the body of the instrument according to the invention, a plate spring coil-shaped stent, multiple spiral stent, deformed tube-shaped stent and the like are applicable. Moreover, a stent body which is in the form of a cylinder by spirally bending an elastic plate member is shown in Figs. 2A and 2B of Japanese Patent Publication No. Hei 4-68939. Although no notch is formed at side surfaces of the cylindrical body, a stent of a cylindrical form arranged as being capable of expanding and contracting along a radial direction of the cylindrical body may also be applicable to as the instrument body of the invention.

The term "stent" used herein includes a covered stent (i.e. those stents wherein a cover is attached on a meshwork or coil-shaped stent or a stent made of a metal formed with a number of holes by means of a laser).

The materials for the stent body is polymer materials, which should have the capability of fixing peptides described hereinafter directly or through a spacer described hereinafter.

Polymer materials can be broadly classified into two categories of biocompatible polymer materials and biodegradable polymer materials.

For the bidocompatible polymers, limitation is not placed on the type of material provided that it has a degree of biocompatibility. Mention is made, for example, silicones, blends or copolymers of polyether-based urethane and dimethylsilicon, polyurethanes, polyacrylamides, plyethylene oxides, polycarbonates and the like.

For the biodegradable polymers, no limitation is placed thereon so far as they have some degrees of rigidity and elasticity and are biodegradable. Mention is made, for example, polyhydroxybutyric acid, polymalic acid, polya-amino acid, collagen, laminin, heparan sulfate, fibronectin, vitronectin, chondroitin sulfate, hyaluronic acid, or copolymers thereof. More preferably, polylactic acid, polyglycollic acid, polycptolactone, plyethylene succinate, polybutylene succinate are mentioned.

The materials for the stent body should be preferably selected from those mentioned above while taking into account a portion where the material is to be applied or how to expand the stent body. More preferably, biodegradable polymer materials are selected.

Where the stent for used as an instrument body of the invention is formed of a biodegradable polymer material, the following merits or characteristics are noted.
(i) Since biodegradable polymer materials have excellent flexibility, good deliverity of the resultant stent at a target site of a diseased blood vessel is attained. (This characteristic is also recognized when using biocompatible polymer materials.)
(ii) Since biodegradable polymer materials are decomposed within the living body, they are decomposed and disappear after playing a role thereof at the target site. Thus, the indwelling instrument of the invention is advantageous in that where an aneurysm that is newly caused at another site of substantially the same blood vessel is cured, it is relatively easy to place another stent. Thus, it becomes feasible to re-cure or perform a plurality of cures at target site or sites within substantially the same area.
(iii) A stent made of a biodegradable polymer material is smaller in radial force than a metallic stent and has self-expandability. Accordingly, a stent made of a biodegradable polymer material is able to avoid occurrence of chronic inflammation caused by the radial force as is caused on placement of the stent on the inner wall of a blood vessel. More particularly, where the stent of a biodegradable polymer material is applied to as an indwelling instrument body of the invention, there can be provided an intravascular, indwelling instrument that is free of or very small in invasion into the living body. This (i.e. a small radial force) is coincident with the fact that unlike a conventional metallic stent which is placed for expanding a diseased blood vessel such as, for example, coronary arteries of the heart, the stent of a biodegradable polymer material is placed on the inner wall of a normal blood vessel in the vicinity of an opening of an aneurysm, so that it is unnecessary to permit a force of radially expanding the blood vessel to work.

The size of the stent body is appropriately determined depending on the portion where applied. For instance, it is preferred that where the stent is applied to the head, the outer diameter prior to expansion is within a range of from 1.0 to 10.0 mm, with a length being within a range of from 5 to 50 mm.

Where a stent is formed of a biodegradable polymer material for used as an instrument body of the invention, it is preferred to set the thickness of the stent body formed of the biodegradable polymer material, for example, at 0.02 to 0.2 mm, more preferably 0.06 to 0.15 mm. This is for the reason that the decomposition of the biodegradable material is completed after achievement of full endothelial conversion. It will be noted that an appropriate thickness is appropriately determined depending on the type of biodegradable polymer material or the outer diameter of a stent body.

The stent body is formed, for example, according to a method including subjecting polyurethane resin powder to extrusion molding to provide a pipe, and cutting the pipe in the form of a stent by laser beam irradiation. Limitation is not placed on the manner of formation, and ordinarily employed forming methods may be appropriated selected depending on the structure of stent and the type of stent material.

### <Peptide>

Next, the peptides of the indwelling instrument according to the invention are described in detail.

The peptide used for the indwelling instrument of the invention is one that has specific interaction with vascular endothelial precursor cells and allows vascular endothelial precursor cells in tissues or body fluids including blood to be selectively adsorbed on and adhered to the body of the instrument.

The peptides of the invention having specific interaction with the vascular endothelial precursor cells include, for example, peptides having any of amino acid sequences including Arg-Glu-Asp-Val (REDV) (sequence No. 1), Arg-Gly-Asp (RGD) (sequence No. 2) and Tyr-Ile-Gly-Ser-Arg (YIGSR) (sequence No. 3).

The amino acid sequences of these peptides are those which are extracted from amino acid sequences of fibronectin, vitronectin, fibrinogen, or laminin that is a protein capable of joining to integrin of a cell surface acceptor. For instance, REDV (sequence No. 1) was reported by Hubbel et al., (see "Bio/Technology" indicated hereinbefore) in 1991 as an attached ligand peptide existing in III-CS region of fibronectin. Likewise, RGD(sequence No. 2) is a sequence commonly existing as an amino acid sequence related to the attachment or adhesion in acceptor proteins including fibronectin, vitronectin and fibrinogen. Moreover, YIGSR is a sequence extracted from laminin and is an amino acid sequence necessary for binding to a non-integrin laminin acceptor existing in a cell surface layer. Accordingly, these peptides (REDV, RGD, YIGSR) are ones of peptides having specific interaction with vascular endothelial precursor cells.

Although the polymer of peptides having such amino acid sequences is not critical, the polymer is preferred, from the standpoint of stability in sterilizing step and deliverity to a target site, within a range of Mw 382 to 10000, more preferably Mw 500 to 5000 and most preferably Mw600 to 850.

The manner of synthesizing the peptide is not critical, and is appropriately selected from ordinarily employed synthesizing methods (solid phase synthesis, liquid phase synthesis, and biological techniques such as genetic engineering).

Next, the fixing method, amount and form of a peptide having specific interaction with vascular endothelial precursor cells on the indwelling instrument of the invention are now described.

The amount of a peptide fixed on the surface of an instrument body surface of the invention is not critical. The amount is appropriately selected depending on the amount of vascular endothelial precursor cells to be adsorbed on and adhered to the surface of the instrument body. The amount is preferably within a range of 1.0 × 10⁻¹⁴ mol/cm² to 1.0 × 10⁻² mol/cm², more preferably 1.0 × 10⁻¹² mol/cm² to 1.0 × 10⁻⁵ mol/cm², and most preferably 1.0 × 10⁻¹¹ mol/cm² to 1.0 × 10⁻⁸ mol/cm².

The form of fixing of a peptide on the surface of the instrument body according to the invention is not critical and should preferably satisfy the following requirements.
i) The peptide should be favorably fixed as existing on at least one of the surfaces at an intravascular lumen side of the instrument body (i.e. inner side of the instrument body) and at the vascular wall side.
ii) The peptide should be favorably fixed as existing uniformly or unevenly on the surface of the instrument body.
iii) In order to provide a good efficiency of adsorption of vascular endothelial precursor cells on the instrument body, the peptide should be favorably fixed, in larger amounts, on the surface side of the body which comes in contact with a larger amount of blood. This is a requirement limited by the existence of a large amount of vascular endothelial precursor cells in blood.
iv) The peptide may have not only one amino acid sequence, but also several types of amino acid sequences in order that the peptide is so designed as to satisfy required absorbability.
v) For the reason that the peptide needs not being radially adjusted in position when placed at an opening of an aneurysm and should favorably be fixed throughout the radial direction of the indwelling instrument.

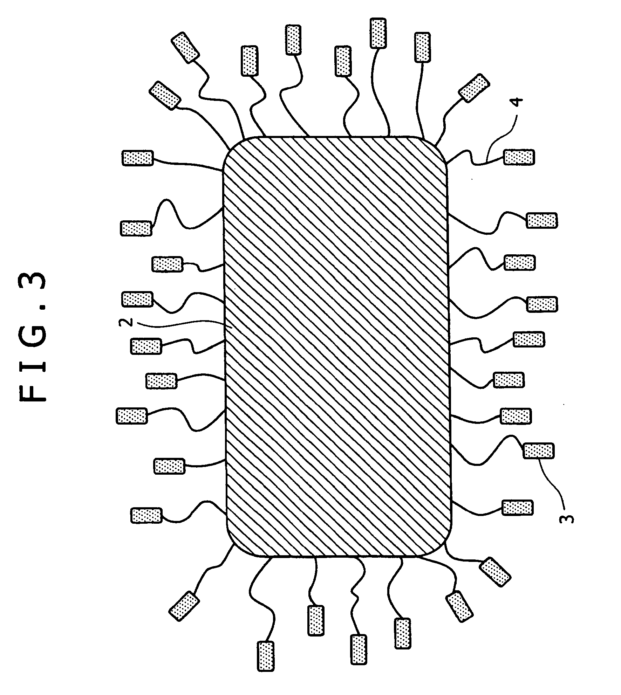

The manner of fixing a peptide on the body surface is not critical. For instance, as shown in Fig. 2, a peptide 3 may be fixed directly on a surface of a stent 2 (direct method). Alternatively, as shown in Fig. 3, the peptide 3 may be attached to the surface such as of the stent 2 through a spacer 4 (indirect method).

The fixing of a peptide on the body surface according to the direct method is realized by covalent linkage or ion linkage. On the other hand, the fixing of a peptide on the surface according to the indirect method is performed with covalent linkage through a spacer made preferably of polyethylene glycol. This is for the reason that non-specific interaction, against the indwelling instrument, with cells or proteins in body fluids including tissues or blood is suppressed, and specific interaction with vascular endothelial precursor cells is likely to proceed.

The spacer used for the fixing of a peptide on the surface of the indwelling instrument body according to the indirect method is polyethylene glycol (PEG), because of its high compatibility to tissues or blood and high mobility of molecular chains. In view of the deliverity to a target site and specific absorbability, PEG should preferably have a polymer of Mw10 to 30 kD, more preferably 15 to 25 kD.

In either of the direct method or indirect method, the fixing of a peptide on the body surface may be carried out by a case where no surface treatment is effected, but relying on covalent linkage or ion linkage, or a case where the surface has been covered beforehand with a surface treating agent such as a silane coupling agent or a polymer to provide a number of functional groups, and the fixing is effected through the functional groups.

The polymers used to cover the body surface should not have functional groups initially. If such functional groups are absent, functional groups may be introduced at a later stage by surface treatments such as a plasma treatment, a corona discharge and the like.

When a peptide is bound to a spacer fixed to a surface or outer surface of the instrument body of the invention, an amino acid is added to the peptide at both ends thereof so as to prevent the peptide having specific interaction with vascular endothelial precursor cells from being damaged. The amino acid to be added is not critical with respect to the type, and preferably includes glycine or tyrosine.

For instance, with a spacer having a calboxyl group or a hydroxyl group at terminal ends, hydroxyl groups are introduced into the surface of the instrument body of the invention. The hydroxyl group is reacted with the carboxyl group of the spacer to form an ester linkage. Thereafter, the hydroxyl group at one terminal of the spacer is reacted with the carboxyl group at a terminal of the peptide to which the amino acid has been added at both terminal ends thereof to form ester linkages. Thus, the spacer allows the peptide and the surface to be fixed through the spacer. With a spacer having a carboxyl group at both terminal ends, after amido linkage with a peptide, another carboxyl group of the spacer and the hydroxyl group at the surface are reacted to provide ester linkage for fixing.

For example, where the instrument body in the blood vessel is made of a polymer such as polylactic acid, a carboxyl group or hydroxyl group is formed through hydrolysis, followed by fixing through ester linkage or covalent linkage through amide linkage.

Next, the description will be made for the insertion and placement method of an intravascular, indwelling instrument of the invention in a target site of a diseased blood vessel.

The instrument is percutaneously inserted by means of a catheter without resorting to a surgical operation. For the manner of placement after the insertion, mention is made of a method wherein the instrument body is a stent, the stent is folded back on itself in a fine and small fashion to remove the force involved therein, so that the stent can be radially expanded by its restoring force (self-expansion type), and a method wherein a stent itself is radially expanded from the inside thereof by means of a balloon (balloon-assisted expansion type). However, the placement is not limited to these methods.

Next, specific effects attained by application of the intravascular, indwelling instrument of the invention to an aneurysm are described.

Where the indwelling instrument is applied to a brain aneurysm having a wide opening without use of an embolizing substance, the instrument is able to facilitate the endothelial conversion of the area at the opening of the aneurysm. On the contrary, if a coil-shaped embolizing substance is used, there is a possibility that the substance withdraws from the aneurysm, and thrombus is formed on the thus withdrawn substance, followed by dispersing the thrombus entrained with a bloodstream into the peripheries. This may eventually bring about complications such as brain infarction. The instrument of the invention can prevent such complications. In this connection, however, if the indwelling instrument is applied to a brain aneurysm having a wide opening along with an embolizing substance in the form of a coil or particles, the indwelling instrument can effectively block withdrawal of the embolizing substance, and therefore, the problem on the withdrawal of the embolizing substance can be solved. Thus, such complications as mentioned above can be prevented.

Further, when the indwelling instrument of the invention, which is able to facilitate the organization of an aneurysm, is applied to a brain aneurysm having a large diameter, the following problem as will be involved, for example, in the use of a coil-shaped embolizing substance can be mitigated: recurrent bleeding may occur due to coil compaction of the coil-shaped embolizing substance filled in the aneurysm after operation or due to the regrowth of aneurysm.

Further, if the indwelling instrument is applied to a brain aneurysm formed at a bifurcation of a blood vessel, the problem involved in the use of a coil-shaped embolizing substance, i.e. there may be a risk of blocking up the bifurcation with the coil-shaped embolizing substance, can be avoided because the instrument of the invention is able to facilitate the organization of the aneurysm without use of an embolizing substance. In this connection, however, if the indwelling instrument of the invention is applied to a brain aneurysm formed at a bifurcation of a blood vessel along with an embolizing substance in the form of a coil or particles, the withdrawal problem of the embolizing substance can be solved, and the instrument can synergistically facilitate the organization of the aneurysm, so that the blocking at the bifurcation as set out above can be inhibited.

In this way, when the indwelling instrument that contains a peptide having specific interaction with vascular endothelial precursor cells is applied to different forms of aneurysms, all or part of the maintained blood flow contact face and the non-maintained blood flow contact face of the instrument body are covered with vascular endothelial precursor cells without using the embolizing substance. This enables facilitation of endothelial conversion of a target site and the organization of the aneurysm. Accordingly, the indwelling instrument ensures a stable cure irrespective of the form of aneurysm. Moreover, if the indwelling instrument is applied to along with a coil-shaped or particulate embolizing substance, the problem on the withdrawal of an embolizing substance can be solved, with the possibility that organization of aneurysm can be synergistically promoted.

The application of the indwelling instrument of the invention to an aneurysm allows vascular endothelial precursor cells to be rapidly bound to all or part of the maintained blood flow contact face or non-maintained blood flow contact face of the instrument. Therefore, endothelial conversion at an opening of an aneurysm can be realized within so short a time as two days for example. This reduces or inhibits a blood flow not to be maintained from entering into the aneurysm, thereby facilitating the organization of the aneurysm. As a consequence, secondary symptoms (subarachnoid bleeding, numbness, anemia and the like) caused by aneurysm formation can be prevented.

The invention is described in more detail by way of examples, which should not be construed as limiting the invention thereto.

### Example 1

A cyclohexyl-based polyurethane powder having a weight-average molecular weight (Mw) of about 200,000 was formed into a pipe having an outer diameter of about 2.0 mm, a thickness of about 150 µm, and a length of 10 mm by means of Laboplast Mill (75C100. made by Toyo Seiki Seisakusho, Ltd.). This pipe was cut into a stent piece by use of an excimer laser (SPL 400H, made by Sumitomo Heavy Industries, Ltd.).

The hydrogen atom of the urethane linkage was withdrawn from the polyurethane, followed by reaction with the carboxyl group of commercially available polyethylene glycol (PEG) modified at ends thereof with a carboxyl group and a hydroxyl group and having a polymer of 20 kD thereby forming an ester linkage. Thereafter, the one end terminal hydroxyl group was reacted with a carboxyl group of an oligopeptide GREDVY (sequence No. 4) wherein glycine (G) and tyrosine (Y) were added to Arg-Glu-Asp-Val (REDV) (sequence No. 1) at both ends thereof to form an ester linkage. Thus, the oligopeptide was fixed, in an amount of 1 µmol per unit stent, on the surface of the stent through covalent linkage using PEG as a spacer.

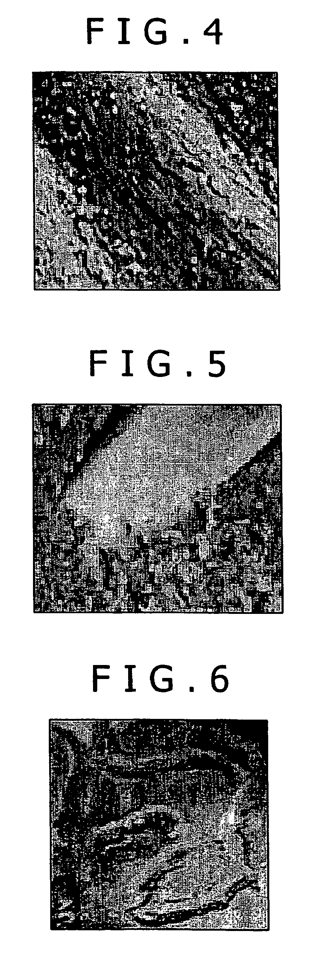

This stent was embedded in the carotid artery of a rabbit artificially induced with aneurysm. As shown in Fig. 4 (an inner surface of a blood vessel of the carotid artery of the rabbit, magnification: 300 x), significant endothelial conversion was recognized.

### Comparative Example 1

For a comparative experiment of Example 1, a stent fixed with no peptide was used, followed by carrying out a similar experiment as in Example 1.

As a result, as shown in Fig. 5 (an inner surface of a carotid-arterial vessel of the rabbit wherein a neck portion of the aneurysm is observed at the reverse print area, magnification: 300 x), significant endothelial conversion was not recognized.

### Example 2

Pellets of a copolymer of polylactic acid-polyglycolic acid fixed with a hydroxy group at terminal ends thereof and having a weight average molecular weight (Mw) of about 100,000 were formed into a pipe having an outer diameter of about 2.0 mm, a thickness of 150 µm and a length of 10 mm by means of Laboplast Mill (75C100, made by Toyo Seiki Seisakusho, Ltd.). This pipe was cut into a stent piece by means of an excimer laser (SPL400H, made by Sumitomo Heavy Industries, Ltd.).

This carboxyl group of commercially available polyethylene glycol (PEG) modified with a carboxyl group and a hydroxyl group at ends thereof and having a polymer of 20 kD were reacted with each other to form an ester linkage. Thereafter, the one-end terminal hydroxyl group was reacted with a carboxyl group of an oligopeptide GREDVY (sequence No. 4) wherein glycine (G) and tyrosine (Y) were added to Arg-Glu-Asp-Val (REDV) (sequence No. 1) at both ends thereof to form an ester linkage. Thus, the oligopeptide was fixed, in an amount of 1 pmol per unit stent, on the surface of the stent through covalent linkage using PEG as a spacer.

The carotid artery of a rabbit was clogged with a balloon, into which 1001U of elastase was charged thereby inducing the formation of a saccular aneurysm. The stent was embedded at a neck portion of the aneurysm for about two days.

As shown in Fig. 6 (an inner surface of the arterial vessel of a rabbit, magnification 150 x), significant endothelial conversion was recognized.

### Comparative Example 2

For a comparative experiment of Example 2, a stent not fixed with any peptide thereon was used to carry out a similar experiment as in Example 2.

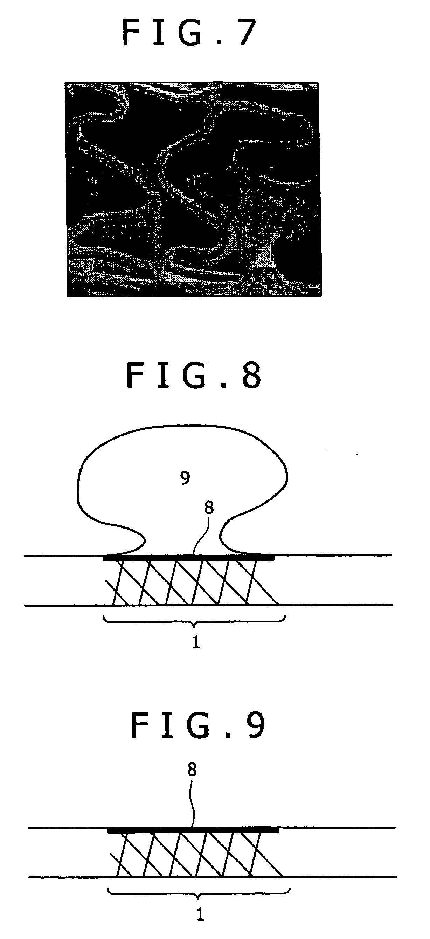

As a result, as shown in Fig. 7 (an inner surface of a carotid-arterial vessel of a rabbit wherein a neck portion of the carotid aneurysm was observed at the reverse print area), significant endothelial conversion was not recognized.

Where the intravascular, indwelling instrument (stent body 1) of the invention is placed at an opening of an aneurysm, vascular endothelial precursor cells are adsorbed on or adhered to the peptide fixed on the surface of the body 1, so that a vascular endothelium 8 is formed (endothelial conversion) so as to block up the opening of the aneurysm 9 as shown in Fig. 8. In this manner, no bloodstream flows in the aneurysm 9, and the organization within the aneurysm 9 is facilitated and a space inside the aneurysm 9 is blocked up. Eventually, the aneurysm 9 disappears as shown in Fig. 9.

## Claims

1. An intravascular, indwelling instrument for placement in a blood vessel, comprising:
a stent made of a high polymer material having an outer surface and an inner surface, and
a peptide fixed by covalent linkage or ion linkage to all or part of the outer or inner surface of the stent and having specific interaction with vascular endothelial precursor cells,
wherein said peptide permits selective adsorption and adhesion of the vascular endothelial precursor cells to cover all or part of the inner surface or the outer surface of the stent with the vascular endothelial precursor cells, **characterized by** the fact that said peptide has an amino acid sequence which is one of Arg-Glu-Asp-Val (REDV) (sequence No. 1), Arg-Gly-Asp (RGD) (sequence No.2) and Tyr-Ile-Gly-Ser-ARg (YIGSR) (sequence No.3).

2. The intravascular, indwelling instrument according to claim 1, wherein said high polymer material consists of a biodegradable polymer.

3. The intravascular, indwelling instrument according to claim 2, wherein said biodegradable polymer consists of at least one member selected from the group consisting of polylactid acid, polyglycolic acid, polycaprolactone, polyethylene succinate, polybutylene succinate, polyhydroxy butyrate, polymalic acid, poly-a-amino acid, collagen, laminin, heparin sulphate, fibronectin, vitronectin, chondroitin sulphate and hyaluronic acid, or a copolymer of monomers for the above-defined biodegradable polymers.

4. The intravascular, indwelling instrument according to any of claim 1 to 4 3, wherein said peptide is fixed by covalent linkage through polyethylene glycol serving as a spacer.

## Patentansprüche

1. Intravasales Verweilinstrument zum Anordnen in einem Blutgefäß, wobei das Instrument Folgendes umfasst:
einen aus einem Hochpolymer-Werkstoff hergestellten Stent, der eine Außenfläche und eine Innenfläche hat,
ein Peptid, das durch eine kovalente Bindung oder eine Ionenbindung an der gesamten Außen- oder Innenfläche des Stents oder einem Teil derselben befestigt ist und eine spezifische Wechselwirkung mit Gefäßendothel-Vorläuferzellen hat,
wobei das Peptid eine selektive Adsorption und Adhäsion der Gefäßendothel-Vorläuferzellen ermöglicht, um die gesamte Innenfläche oder die Außenfläche des Stents oder einen Teil derselben mit den Gefäßendothel-Vorläuferzellen zu bedecken, **gekennzeichnet durch** die Tatsache, dass das Peptid eine Aminosäurefolge hat, die eine der folgenden ist: Arg-Glu-Asp-Val (REDV) (Folge Nr. 1), Arg-Gly-Asp (RGD) (Folge Nr. 2) und Tyr-Ile-Gly-Ser-Arg (YIGSR) (Folge Nr. 3).

2. Intravasales Verweilinstrument nach Anspruch 1, wobei der Hochpolymer-Werkstoff aus einem biologisch abbaubaren Polymer besteht.

3. Intravasales Verweilinstrument nach Anspruch 2, wobei das biologisch abbaubare Polymer aus wenigstens einem Element besteht, das aus der Gruppe ausgewählt ist, die aus Polymilchsäure, Polyglykolsäure, Polycaprolacton, Polyethylensuccinat, Polybutylensuccinat, Polyhydroxybutyrat, Polyäpfelsäure, Poly-a-Aminosäure, Kollagen, Laminin, Heparinsulfat, Fibronektin, Vitronektin, Chondroitinsulfat und Hyaluronsäure oder einem Copolymer von Monomeren für die weiter oben definierten biologisch abbaubaren Polymere besteht.

4. Intravasales Verweilinstrument nach einem der Ansprüche 1 bis 3, wobei das Peptid durch eine kovalente Bindung befestigt ist, wobei Polyethylenglykol als Spacer dient.

## Revendications

1. Instrument intravasculaire à demeure, à mettre en place dans un vaisseau sanguin, comprenant :
- un stent en un matériau de type haut polymère, présentant une surface externe et une surface interne,
- et un peptide fixé, par liaison covalente ou par liaison ionique, sur tout ou partie de la surface interne ou externe du stent, lequel peptide peut interagir de façon spécifique avec les cellules précurseurs de cellules endothéliales vasculaires,
lequel peptide permet l'adsorption et l'adhésion sélectives des cellules précurseurs de cellules endothéliales vasculaires, de sorte que ces cellules précurseurs de cellules endothéliales vasculaires recouvrent tout ou partie de la surface interne ou de la surface externe du stent,
**caractérisé en ce que** ledit peptide présente une séquence d'acides aminés qui est l'une des suivantes :
- Arg-Glu-Asp-Val ou REDV (séquence N° 1) ;
- Arg-Gly-Asp ou RGD (séquence N° 2) ;
- et Tyr-Ile-Gly-Ser-Arg ou YIGSR (séquence N° 3).

2. Instrument intravasculaire à demeure, conforme à la revendication 1, dans lequel ledit matériau de type haut polymère est un polymère biodégradable.

3. Instrument intravasculaire à demeure, conforme à la revendication 2, dans lequel ledit polymère biodégradable consiste en au moins un élément de l'ensemble formé par les suivants : poly(acide lactique), poly(acide glycolique), polycaprolactone, poly(éthylène succinate), poly(butylène succinate), poly(hydroxy-butyrate), poly(acide malique), poly(alpha-amino-acide), collagène, laminine, héparine-sulfate, fibronectine, vitronectine, chondroïtine-sulafte et acide hyaluronique, ou en un copolymère des monomères des polymères biodégradables mentionnés ci-dessus.

4. Instrument intravasculaire à demeure, conforme à l'une des revendications 1 à 3, dans lequel ledit peptide est fixé par liaison covalente, par l'intermédiaire d'un polyéthylène-glycol qui sert d'espaceur.
